# EUROPÄISCHE PATENTSCHRIFT

(11) **EP 0 773 739 B1**
(45) Veröffentlichungstag und Bekanntmachung des Hinweises auf die Patenterteilung: **12.12.2001**
(21) Anmeldenummer: 95927630.4
(22) Anmeldetag: 04.08.1995
(51) Int. Cl.: A61B 6/14

(54) **ZAHNÄRZTLICHE RÖNTGENDIAGNOSTIKEINRICHTUNG**
DENTAL X-RAY DIAGNOSTIC DEVICE
DISPOSITIF DE RADIODIAGNOSTIC DENTAIRE

(30) Priorität: 05.08.1994 DE 4427858
(43) Veröffentlichungstag der Anmeldung: 21.05.1997
(73) Patentinhaber: Sirona Dental Systems GmbH, 64625 Bensheim (DE)
(72) Erfinder: EICHHORN, Gerd, D-68623 Lampertheim (DE); REINKE, Michael, D-64289 Darmstadt (DE); SCHRICK, Klaus, D-64625 Bensheim (DE); SCHULZE-GANZLIN, Ulrich, D-64653 Lorsch (DE)
(74) Vertreter: Sommer, Peter
(86) Internationale Anmeldenummer: DE9501023
(87) Internationale Veröffentlichungsnummer: WO9603917

(56) Entgegenhaltungen:
- EP-A- 0 285 214
- EP-A- 0 544 974
- DE-U- 8 909 398
- FR-A- 2 596 603
- FR-A- 2 699 805
- US-A- 4 823 809
- US-A- 5 058 045
- US-A- 5 319 751
- US-A- 5 365 069
- LASER FOCUS WORLD, Bd. 27, Nr. 6, Juni 1991 TULSA, US, Seiten 111-115, CARTS 'Solid-state cameras focus on high-end uses'

## Beschreibung

Die Erfindung betrifft eine zahnärztliche Röntgendiagnostikeinrichtung zur Erstellung von intra- oder extraoralen Röntgenaufnahmen, umfassend einen Röntgenstrahler, der am einen Ende eines vorzugsweise mehrgliedrigen Tragarmes angeordnet ist, dessen anderes Ende an einem Wandadapter angelenkt ist, eine mit Bildschirm ausgestattete Recheneinheit, einen extra-oder intraoral eines Patientenmundes plazierbaren strahlenempfindlichen Sensor mit Röntgenbildaufnehmer, eine Elektronik zur Ansteuerung des Strahlers, des Sensors sowie zur Datenübertragung, wobei die vorgenannten Komponenten kommunikativ miteinander in Verbindung stehen.

Eine Röntgendiagnostikeinrichtung mit extraoral eines Patientenmundes plazierbarem Sensor ist beispielsweise in der DE-43 22 483 beschrieben. Mit der in diesem Dokument offenbarten Einrichtung lassen sich sowohl Panorama-Aufnahmen vom Kiefer eines Patienten als auch Schädelaufnahmen (CEPH-Aufnahmen) erstellen.

Ein intraoral eines Patientenmundes plazierbarer Sensor ist in der DE-43 03 939 offenbart. Der Sensor ist vorteilhafterweise ein CCD-Sensor, der in der Regel über ein Verbindungskabel mit einer Recheneinheit verbunden ist, in der die vom Sensor gewonnenen Bilddaten ausgewertet und als elektrische Singale einem Bildschirm zugeführt werden.

Eine ähnliche Röntgendiagnostikeinrichtung ist aus FR-A-25 96 603 bekannt. Der CCD-Sensor ist dort über in Interface mit einer Recheneinheit verbunden, die mehrere Elektronikkarten zur Bildaufbereitung und Bildwiedergabe enthält.

Nachteilig bei einer solchen Einrichtung ist, daß das gesamte Kommunikationssystem hardwaremäßig aufeinander abgestimmt sein muß, was bedeutet, daß die Recheneinheit so konzipiert sein muß, daß sie zum eingesetzten Sensor bzw. vorgesehenen Röntgengerät paßt.

Dem Anspruch 1 der angegebenen Erfindung liegt die Aufgabe zugrunde, diesen Nachteil zu vermeiden und eine Röntgendiagnostikeinrichtung anzugeben, mit der es möglich ist, intra-oder extraorale Aufnahmen unter Verwendung eines PC erstellen zu können.

Die Aufgabe der Erfindung wird mit den Merkmalen des Anspruchs 1 gelöst.

Eine Ausführung der Erfindung sieht vor, die Recheneinheit, also den PC, mit einer Aufnahmevorrichtung für eine von außen an den PC anzuschließende Röntgenbilder-fassungskarte zu versehen. Diese Erfassungskarte ist vorteilhafterweise als PCMCIA-Interface-Karte (gefördert von Personal Computer Memory Card International Association)ausgebildet oder vorteilhafterweise mit Mitteln zur drahtlosen Datenübertragung versehen. Durch diese Maßnahmen lassen sich Röntgenaufnahmen erstellen, ohne daß ein Eingriff in den PC erforderlich ist. Ohne diese Maßnahmen wäre es bei Verwendung eines PC als Recheneinheit erforderlich, für die Erfassungskarte, die zumindest die Ansteuerelektronik für den CCD-Sensor und gegebenenfalls diverse Speicher sowie einen Grafikprozessor zur Bildverarbeitung enthält, einen separaten Steckplatz im PC bereitzustellen. Für einen Wechsel bzw. einen Austausch der im PC eingesteckten Erfassungskarte wäre es außerdem notwendig, den PC zu öffnen.

Ein besonders mobiles und flexibel einsetzbares Röntgensystem wird geschaffen, wenn man als Recheneinheit ein Laptop oder Notebook vorsieht. Das System ist transportabel, kann zur Darstellung von Röntgenaufnahmen genutzt werden und kann auch zur Unterstützung des Patientengesprächs dienen. Sofortaufnahmen können vor Ort ohne Hilfsmonitor erstellt werden. Der PC kann über Akku betrieben werden, so daß keine aufwendigen Schutzmaßnahmen für den Patienten gegenüber Netzspannung nötig sind. Es brauchen somit keine Maßnahmen für eine galvanische Trennung der Netzspannung führenden Teile getroffen zu werden.

Alternativ zu der oben angesprochenen Ausführung, bei der Sensor und Röntgenbilderfassungskarte von außen, also als externe Komponente an den PC angeschlossen werden, kann es vorteilhaft sein, die Ansteuerelektronik und die Mittel für die Datenübertragung zu integrieren. Insbesondere bei Röntgendiagnostikeinrichtungen, bei denen der Röntgenstrahler mittels eines Tragarmes an einem Wandadapter angelenkt ist, kann es vorteilhaft sein, den Sensor am Wandadapter zu haltern und die Ansteuerelektronik sowie die Mittel zur Datenübertragung im Wandadapter unterzubringen.

Alternativ können der Sensor und Teile der Elektronik am bzw. im Gehäuse des Röntgenstrahlers selbst untergebracht sein. Letztere Variante hat den Vorteil, daß das Verbindungskabel zum Sensor nur sehr kurz zu sein braucht. Vorteile beider Varianten sind, daß die Steuerung der Bilderfassung über die Steuerung des Röntgengerätes erfolgen kann und somit keine Strahlendetektion notwendig ist.

Da beide Varianten auch mit einem primären Bildspeicher ausgestattet sein können, ist es vorteilhaft, über ein im Wandadapter untergebrachtes Display Röntgenbilder zur Kontrolle der vorhergehenden Aufnahmen darzustellen. Diese Darstellung kann unabhängig vom angeschlossenen PC erfolgen, der sich damit vorteilhafterweise auch außerhalb des Röntgenortes befinden kann.

Anhand der Zeichnung werden nachfolgend mehrere Ausführungsbeispiele der Erfindung näher erläutert.

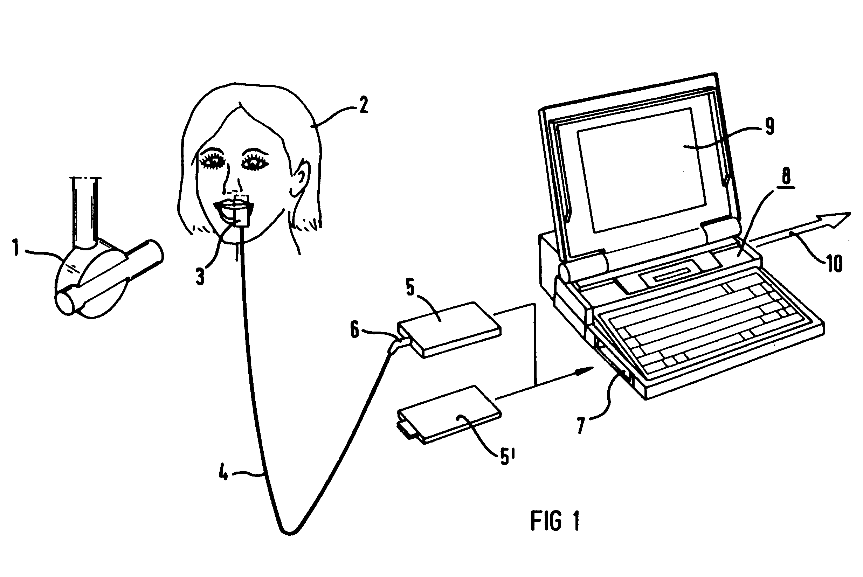

Die Figur 1 zeigt in einer schaubildlichen Darstellung eine zahnärztlicheRöntgendiagnostikeinrichtung zur Erstellung von Zahn- oder Kieferaufnahmen eines Patienten, mit einem Röntgenstrahler 1 und einem intraoral eines Patientenkopfes 2 plazierbaren strahlenempfindlichen Sensor 3, der vorteilhafterweise als CCD-Sensor ausgebildet ist. Der Sensor 3 ist über ein Kabel 4 mit einer Röntgenbilderfassungskarte 5 verbunden, die mit PCMCIA-Interface ausgebildet ist. Das Sensorkabel 4 ist vorteilhafterweise mittels eines Steckanschlusses 6 an der Erfassungskarte 5 befestigt, wodurch sich u.a. die Desinfektion des Sensors leichter durchführen läßt.

Eine Steckverbindung kann auch unmittelbar am Sensor oder innerhalb des Sensorkabels vorgesehen werden. Dies würde eine Desinfektion erleichtern, da nicht das gesamte Kabel desinfiziert werden muß.

Die Röntgenbilderfassungskarte 5 ist, wie durch Pfeil angegeben, an eine entsprechend ausgebildete Aufnahmevorrichtung 7 eines Laptops 8 anschließbar. Das Laptop, das in bekannter Weise einen Bildschirm 9 enthält, ist vorteilhafterweise unabhängig vom Versorgungsnetz ausgebildet, d.h. es wird mit Batterie oder Akku betrieben, wodurch eine zusätzliche galvanische Trennung zwischen Sensor und Rechner entfallen kann. Gegebenenfalls kann das Laptop mit Komponenten zur drahtlose Datenübertragung versehen sein und an ein bestehendes Rechnernetz ange bunden werden, wie dies durch den Pfeil 10 angedeutet ist.

Durch die drahtlose Datenübertragung lassen sich folgende Vorteile erzielen:
- Gegenüber bestehenden Zahnarzt-EDV-Anlagen ist eine einfache und eindeutige Schnittstelle geschaffen, die relativ einfache Integrations- und Anbindungsmögmöglichkeiten bietet.
- Zentrale Einrichtungen, wie Server, Drucker, etc., können besser genutzt werden.
- Eine Datenübertragung ist direkt am PC oder Netz (Ethernet oder Token Ring) möglich.
- Innerhalb der Praxis ist ein mobiler Einsatz des Röntgen-PC möglich.

Alternativ zu der vorbeschriebenen Ausführung der Röntgenbilderfassungskarte 5 kann diese mit geeigneten Mitteln zur drahtlosen Übertragung der Daten vom Sensor 3 in die Recheneinheit (Laptop 8) versehen sein. Geeignete Mittel zur drahtlosen Übertragung können die an sich bekannten Infrarotsende- und -empfangseinrichtungen sein.

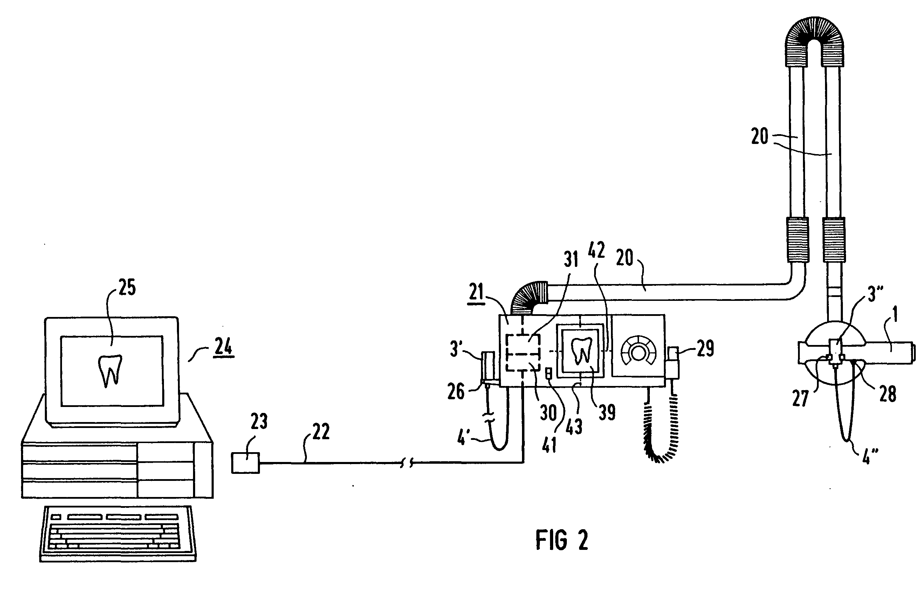

Anhand der Figur 2 werden zwei weitere vorteilhafte Ausführungen beschrieben.

Die Röntgendiagnostikeinrichtung nach der Figur 2 umfaßt ein zahnärztliches Röntgengerät, bei dem der Röntgenstrahler 1 mittels eines mehrgliedrigen Tragarmes 20 an einem Wandadapter 21 angelenkt ist. Das Röntgengerät ist mittels eines Kabels 22 mit Stecker 23 mit einem PC 24 mit Monitor 25 verbindbar. Der PC ist bei diesem Ausführungsbeispiel - im Gegensatz zu dem nach Figur 1 - eine Desktop-Ausführung. In einer ersten vorteilhaften Variante ist der Sensor (3') mittels einer Halterung 26 am Gehäuse des Wandadapters 21 gehaltert. Der Vorteil dieser Anordnung ist, daß Tragarm und Strahler unverändert bleiben können.

In einer zweiten Ausführung ist der Sensor (3") mittels einer Halterung 27 am Gehäuse des Röntgenstrahlers 1 gehaltert. Der Vorteil dieser Anordnung ist, daß die Länge des Sensorkabels (4") wesentlich kürzer als bei den anderen Varianten ausgeführt zu sein braucht, da der Sensor nur bis kurz vor dem Strahlertubus reichen muß (sh. Fig. 1).

Vorteilhaft ist es, den Anschluß des Sensorkabels 4 an die Elektronik als leicht trennbaren Anschluß, vorzugsweise als Steckanschluß (in Fig. 2 mit der Pos. 28 angedeutet) auszubilden; es bietet sich so die Möglichkeit, mehrere Sensoren unterschiedlicher Größen alternativ anzuschließen. Denkbar ist es auch, den Anschluß zwischen Sensor und Sensorkabel als Steckanschluß auszubilden.

In einer optionalen Ausbauvariante ist dem Wandadapter 21 ein Flach-Display 39 zugeordnet, welches Röntgenbilder zur Kontrolle vorhergehender Aufnahmen darstellt. Dies ist zweckmäßig, um gegebenenfalls Fehlpositionierungen bei der Anwendung des Sensors zu erkennen. Dieses Display kann starr in der Front des Wandadapters eingelassen sein oder, je nach Aufstellungsort des Gerätes zum Betrachter, um eine horizontale und/oder vertikale Achse 42, 43 schwenk- bzw. kippbar gelagert sein, wie dies in Figur 2 angedeutet ist. Für den Anwender lassen sich somit ergonomisch günstigere Betrachtungsbedingungen erzielen. Es ist auch vorstellbar, daß das Display gemeinsam mit dem optionalen Fernauslöser 29 des Röntgengerätes abseits des Wandadapters und außerhalb des Röntgenraumes untergebracht wird. In diesem Fall müßte für eine entsprechende Kabelverbindung zwischen Wandadapter und Display gesorgt werden. Die Auswahl vorhergegangener Röntgenbilder erfolgt mit einem am Wandadapter angeordneten Bedienelement 41, das als Plus/Minustaste oder Drehknopf ausgestaltet werden kann. Weitere Bedienelemente können für die Einstellung von Helligkeit und Kontrast vorgesehen werden.

Im folgenden wird die elektrische bzw. elektronische Verknüpfung der Komponenten für die einzelnen Varianten näher beschrieben.

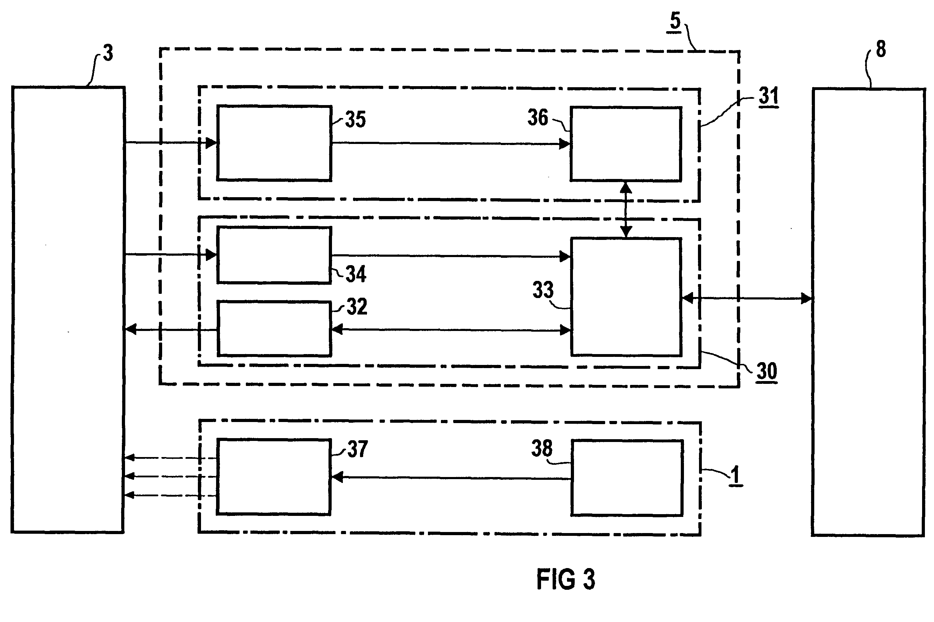

Die Figur 3 zeigt die Verknüpfung der Komponenten für die Ausführungsform nach Figur 1. Der Sensor 3 ist über nicht näher bezeichnete Steuer- bzw. Übertragungsleitungen, die im Sensorkabel 4 geführt sind, mit der Röntgenbilderfassungskarte (RBE) 5 und diese wiederum mit dem PC (Laptop 8) verbunden.

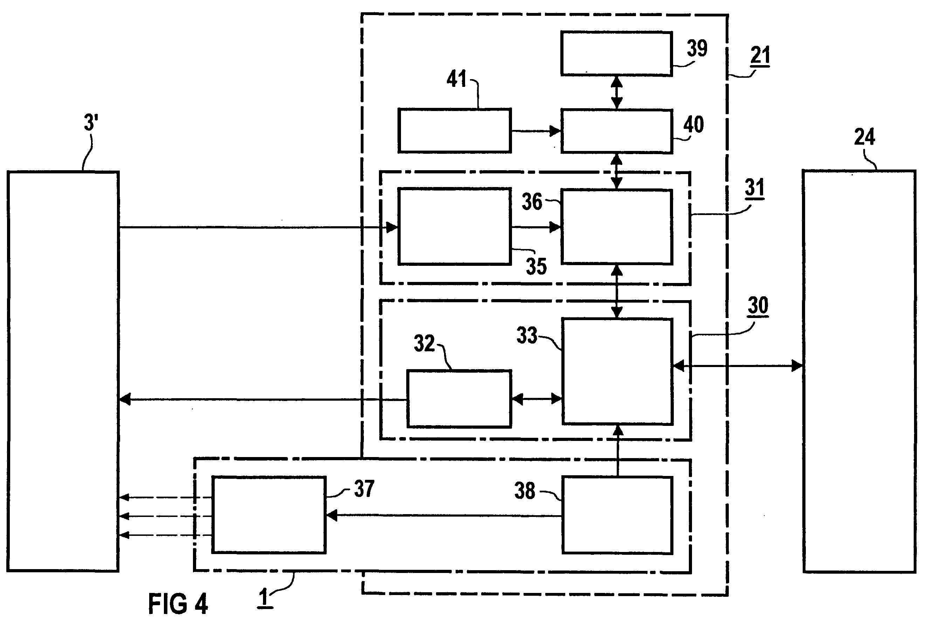

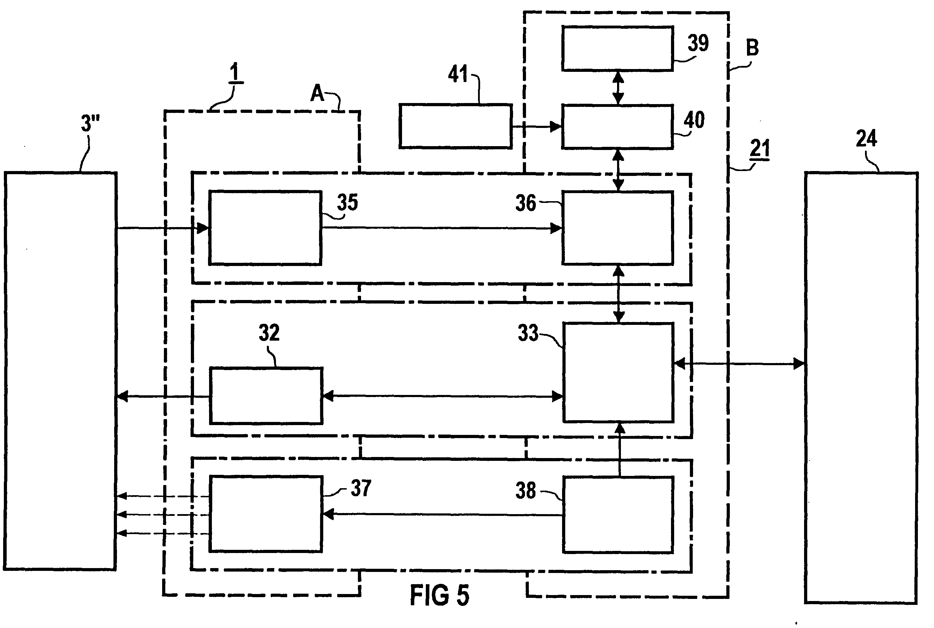

Die RBE umfaßt eine Ansteuerelektronik 30 (CONT-E) und Datenübertragungsmittel (DAT-E) 31. Die Ansteuerelektronik 30 enthält Mittel 32 zur Ansteuerung des Sensors 3, d.h. Mittel zur Bereitstellung der Betriebsspannungen und Steuersignale für den Sensor. Weiterhin enthält sie Mittel 33 zur Steuerung der Bilderfassung. Diese Funktionsgruppe übernimmt die Steuerung aller Funktionen und ist sowohl mit dem Röntgengerät als auch mit dem PC verbunden (sh. auch Fig. 4 und 5).

Als Standard-Schnittstelle zum PC stehen nachfolgend angeführte Ausführungsbeispiele zur Diskussion, wobei diese Schnittstellen unabhängig von der PC-Art sind, also unabhängig davon, ob ein stationäres Desktop-Modell oder eine mobile Ausführung zur Anwendung kommt:
- PCMCIA (Personal Computer Memory Card International Association)
- SCSI (Small Computer System Interface)
- USB (Universal Serial Bus u.a. von NEC, Intel, Microsoft)

Die Ansteuerelektronik 30 enthält noch Mittel 34 zur Strahlendetektion, die bewirken, daß der Sensor in den Zustand versetzt wird, Röntgenstrahlen zu integrieren. Dazu benötigt dieser einen Triggerimpuls, der den Strahlungsbeginn anzeigt. Dieses Signal kann vom Röntgengerät selbst stammen. Falls jedoch auf diese Verbindung zum Röntgengerät verzichtet wird, muß eine eigenständige Funktionsgruppe dieses Signal detektieren.

Die Datenübertragungsmittel 31 umfassen einen A/D-Wandler 35 und einen primären Bildspeicher 36. Der primäre Bildspeicher 36 ist ein Zwischenspeicher, in dem das Röntgenbild komplett oder in Teilen erstmalig digital abgespeichert wird, bevor es durch Bildverarbeitung optimiert und/oder sukzessiv auf einen endgültigen Speicher (z.B. einen Speicher im PC) übertragen wird.

Das in Figur 2 gezeigte, dem Wandadapter zugeordnete Display 39 ist bidirektional mit einer Steuerung 40 verbunden, die wiederum mit dem Bildspeicher 36 zusammenwirkt. Je nach Ausbaugröße des primären Bildspeichers 36 lassen sich mit Hilfe einer Display-Steuerung 40 die letzten Aufnahmen zu Kontrollzwecken darstellen. Das Display wird über die Display-Steuerung, die die bereits erwähnten Bedienelemente 41 aufweist, vom Anwender eingestellt.

Die Strahlenquelle 37 des Röntgenstrahlers 1 wird von einer Steuereinheit 38 angesteuert; diese kann auch extern des Strahlers 1, z.B. im Wandadapter 21 (Fig. 2) angeordnet sein.

Die Figur 4 zeigt die Verknüpfung entsprechend der ersten Ausführungsform nach Figur 2, bei der die gesamte Elektronik, also die Ansteuerelektronik 30 und die Datenübertragungsmittel 31 im Wandadapter 21 angeordnet sind. Bei dieser Version ist keine Strahlendetektion notwendig, was den Vorteil hat, daß eine optimale Bildqualität auch bei extrem kurzen oder langen Belichtungszeiten erreichbar ist. Das Sensorkabel (4') verläuft hier außerhalb des Tragarmes 20 im Gegensatz zu der zweiten Variante, bei der, wenn die Elektronik im Gehäuse des Strahlers 1 untergebracht ist, das Sensorkabel wesentlich kürzer ausgeführt zu sein braucht.

Die Figur 5 läßt die Verknüpfung erkennen, wobei durch die gestrichelte Linienführung angedeutet ist, daß ein Teil (A) der gesamten Elektronik im oder am Strahler 1 und der andere Teil (B) im Wandadapter angeordnet sein kann.

Im Rahmen der Erfindung sind noch folgende weitere Varianten möglich:
Der primäre Bildspeicher 36 kann gegebenenfalls auch im PC untergebracht werden. Dies reduziert die Kosten, jedoch leidet die Datenübertragungssicherheit, da keine asynchrone Datenübertragung zwischen Sensor und PC garantiert werden kann. Teile der Elektronik können auch dem Sensor zugeordnet werden, die entweder im Stecker oder direkt im Sensor am Strahlendetektor untergebracht werden. Dies betrifft im wesentlichen:
   den Vorverstärker, den A/D-Wandler, die Strahlendetektion, die Ansteuerung des Sensors sowie eine elektrisch lesbarer Seriennummer zur Sensoridentifikation.

Das Verlagern von Elektronik in Richtung Sensor ist dann von Vorteil, wenn dieser gegen andere Sensortypen austauschbar ist und in der Elektronik sich alle sensortypspezifischen Eigenschaften befinden. Damit ist eine einheitliche Schnittstelle zwischen verschiedenen Sensortypen und dem restlichen System möglich. Weitere Angaben, z.B. über den Sensortyp oder die Sensorversion, sind über eine elektrisch auslesbare Seriennummer zur Sensoridentifikation indirekt abrufbar.

## Patentansprüche

1. Zahnärztliche Röntgendiagnostikeinrichtung zur Erstellung von intra- oder extraoralen Röntgenaufnahmen, umfassend einen Röntgenstrahler (1), der am einen Ende eines vorzugsweise mehrgliedrigen Tragarmes (20) angeordnet ist, dessen anderes Ende an einem Wandadapter (21) angelenkt ist, eine mit Bildschirm (9, 25) ausgestattete Recheneinheit (8, 24), einen extra- oder intraoral eines Patientenmundes plazierbaren strahlenempfindlichen Sensor (3, 3', 3") mit Röntgenbildaufnehmer, eine Elektronik (30, 31, 38) zur Ansteuerung des Strahlers (1), des Sensors (3, 3', 3'') sowie zur Datenübertragung, wobei die vorgenannten Komponenten kommunikativ miteinander in Verbindung stehen, **dadurch gekennzeichnet, daß** derjenige Teil der Elektronik (30, 31), der zur Ansteuerung des Sensors (3') und zur Datenübertragung dient, zu einer Röntgenbilderfassungskarte (RBE) zusammengefaßt ist und die Röntgenbilderfassungskarte von außen über eine Standard-Schnittstelle mit der Recheneinheit (8, 24) verbunden ist.

2. Röntgendiagnostikeinrichtung nach Anspruch 1, **dadurch gekennzeichnet, daß** die Elektronik (31) zur Datenübertragung einen primären Bildspeicher (36) umfaßt, der mit Mitteln (33) zur Bilderfassung verbunden ist.

3. Röntgendiagnostikeinrichtung nach Anspruch 1, **dadurch gekennzeichnet, daß** die Recheneinheit (8) mit einer Aufnahmevorrichtung (7) für den externen Anschluß der Bilderfassungskarte (5, 5') versehen ist.

4. Röntgendiagnostikeinrichtung nach Anspruch 3, **dadurch gekennzeichnet, daß** die Aufnahmevorrichtung (7) zum Anschluß einer PCMCIA-Interface-Karte ausgebildet ist.

5. Röntgendiagnostikeinrichtung nach Anspruch 1, **dadurch gekennzeichnet, daß** die Röntgenbilderfassungskarte (RBE) im Gehäuse des Wandadapters (21) angeordnet ist und der Sensor (3') am Wandadapter (21) abnehmbar gehaltert ist.

6. Röntgendiagnostikeinrichtung nach Anspruch 1, **dadurch gekennzeichnet, daß** zumindest derjenige Teil (A) der Elektronik (30, 31), der zur Ansteuerung des Sensors sowie zur Datenübertragung dient, im Gehäuse des Röntgenstrahlers (1) untergebracht ist.

7. Röntgendiagnostikeinrichtung nach einem der Ansprüche 1 bis 6, **dadurch gekennzeichnet, daß** die Röntgenbilderfassungskarte (5') Mittel zur drahtlosen Übertragung der Daten vom Sensor (3) zur Recheneinheit (8) enthält.

8. Röntgendiagnostikeinrichtung nach einem der Ansprüche 1 bis 7, **dadurch gekennzeichnet, daß** Sensor (3, 3', 3'') und Sensorkabel (4, 4', 4'') und/oder Sensorkabel (4, 4', 4'') und Röntgenbilderfassungskarte (RBE) mit leicht trennbaren Anschlußkupplungen (28) versehen sind.

9. Röntgendiagnostikeinrichtung nach einem der Ansprüche 1 bis 8, **dadurch gekennzeichnet, daß** eine Netzwerkanbindung (10) mit Standard-Komponenten für eine drahtlose Datenübertragung realisiert ist.

10. Röntgendiagnostikeinrichtung nach einem der Ansprüche 1 bis 9, **dadurch gekennzeichnet, daß** als Recheneinheit (8) ein Laptop oder Notebook verwendet ist.

11. Röntgendiagnostikeinrichtung nach einem der Ansprüche 2 bis 10, **dadurch gekennzeichnet, daß** dem Wandadapter (21) ein Display (39) zur Kontrolle vorangegangener Aufnahmen zugeordnet ist.

12. Röntgendiagnostikeinrichtung nach Anspruch 11, **dadurch gekennzeichnet, daß** das Display (39) schwenk- und/oder kippbar am Wandadapter (21) gehaltert ist.

13. Röntgendiagnostikeinrichtung nach Anspruch 12, **dadurch gekennzeichnet, daß** das Display (39) zusammen mit einer Fernauslöseeinrichtung (29) eine räumlich getrennt vom Wandadapter (21) angeordnete Baueinheit bilden.

## Claims

1. Dental X-ray diagnostic installation for taking intraoral or extraoral X-ray images, comprising an X-ray machine (1), which is arranged at one end of a preferably multipartite supporting arm (20), and whose other end is pivoted to a wall adapter (21), a computing unit (8, 24) equipped with a display screen (9, 25), a radiation-sensitive sensor (3, 3', 3") which can be placed extraorally or intraorally in relation to a patient's mouth and has a X-ray image pickup, an electronic system (30, 31, 38) for driving the machine (1) and the sensor (3, 3', 3'') as well as for data transmission, the abovenamed components being communicatively connected to one another, **characterized in that** that part of the electronic system (30, 31) which serves to drive the sensor (3') and for data transmission is combined to form an X-ray acquisition card (RBE), and the X-ray acquisition card is connected from outside via a standard interface to the computing unit (8, 24).

2. X-ray diagnostic installation according to Claim 1, **characterized in that** the electronic system (31) for data transmission comprises a primary image memory (36) which is connected to means (33) for image acquisition.

3. X-ray diagnostic installation according to Claim 1, **characterized in that** the computing unit (8) is provided with a receptacle device (7) for the external connection of the image acquisition card (5, 5').

4. X-ray diagnostic installation according to Claim 3, **characterized in that** the receptacle device (7) is designed for connecting a PCMCIA interface card.

5. X-ray diagnostic installation according to Claim 1, **characterized in that** the X-ray image acquisition card (RBE) is arranged in the housing of the wall adapter (21), and the sensor (3') is held detachably on the wall adapter (21).

6. X-ray diagnostic installation according to Claim 1, **characterized in that** at least that part (A) of the electronic system (30, 31) which serves to drive the sensor and for data transmission is accommodated in the housing of the X-ray machine (1).

7. X-ray diagnostic installation according to one of Claims 1 to 6, **characterized in that** the X-ray image acquisition card (5') contains means for wireless transmission of the data from the sensor (3) to the computing unit (8).

8. X-ray diagnostic installation according to one of Claims 1 to 7, **characterized in that** the sensor (3, 3', 3'') and sensor cable (4, 4', 4'') and/or sensor cable (4, 4', 4'') and X-ray image acquisition card (RBE) are provided with easily separable connecting couplings (28).

9. X-ray diagnostic installation according to one of Claims 1 to 8, **characterized in that** a network link (10) with standard components is implemented for wireless data transmission.

10. X-ray diagnostic installation according to one of Claims 1 to 9, **characterized in that** a laptop or notebook is used as computing unit (8).

11. X-ray diagnostic installation according to one of Claims 2 to 10, **characterized in that** a display (39) for monitoring preceding images is assigned to the wall adapter (21).

12. X-ray diagnostic installation according to Claim 11, **characterized in that** the display (39) is held pivotably and/or tiltably on the wall adapter (21).

13. X-ray diagnostic installation according to Claim 12, **characterized in that** together with a remote tripping device (29), the display (39) forms an assembly arranged spatially separate from the wall adapter (21).

## Revendications

1. Dispositif de diagnostic à rayons X de dentisterie pour la réalisation de radiographies intra-orales ou extra-orales, comprenant une source de rayons X (1), qui est disposée à une extrémité d'un bras-support (20), de préférence formé de plusieurs tronçons, dont l'autre extrémité est articulée sur un adaptateur mural (21), une unité de calcul (8, 24) pourvue d'un écran de visualisation (9, 25), un capteur (3, 3', 3") sensible au rayonnement, qui peut être placé à l'extérieur ou à l'intérieur de la cavité buccale d'un patient, avec un récepteur d'image radiographique, une électronique (30, 31, 38) pour la commande de la source de rayons (1), du capteur (3, 3', 3") ainsi que pour la transmission de données, lesdits composants étant reliés entre eux de manière communicante, **caractérisé en ce que** la partie de l'électronique (30, 31) servant à la commande du capteur (3') et à la transmission de données est regroupée en une carte d'acquisition d'images radiographiques (RBE) et **en ce que** la carte d'acquisition d'images radiographiques est reliée extérieurement à l'unité de calcul (8, 24) par l'intermédiaire d'une interface standard.

2. Dispositif de diagnostic à rayons X selon la revendication 1, **caractérisé en ce que** l'électronique (31) pour la transmission de données comprend une mémoire d'image primaire (36) qui est connectée à des moyens (33) d'acquisition d'image.

3. Dispositif de diagnostic à rayons X selon la revendication 1, **caractérisé en ce que** l'unité de calcul (8) est équipée d'un dispositif de réception (7) pour la connexion externe de la carte d'acquisition d'image (5, 5').

4. Dispositif de diagnostic à rayons X selon la revendication 3, **caractérisé en ce que** le dispositif de réception (7) est agencé pour la connexion d'une carte d'interface PCMCIA.

5. Dispositif de diagnostic à rayons X selon la revendication 1, **caractérisé en ce que** la carte d'acquisition d'images radiographiques (RBE) est disposée dans le boîtier de l'adaptateur mural (21) et que le capteur (3') est fixé de manière démontable sur l'adaptateur mural (21).

6. Dispositif de diagnostic à rayons X selon la revendication 1, **caractérisé en ce qu'**au moins la partie (A) de l'électronique (30, 31) qui sert à la commande du capteur et à la transmission de données est logée à l'intérieur du boîtier de la source de rayons X (1).

7. Dispositif de diagnostic à rayons X selon une des revendications 1 à 6, **caractérisé en ce que** la carte d'acquisition d'images radiographiques (5') comprend des moyens pour la transmission sans fil des données du capteur (3) à l'unité de calcul (8).

8. Dispositif de diagnostic à rayons X selon une des revendications 1 à 7, **caractérisé en ce que** le capteur (3, 3', 3") et le câble de capteur (4, 4', 4") et/ou le câble de capteur (4, 4', 4") et la carte d'acquisition d'images radiographiques (RBE) sont munis de connecteurs (28) facilement séparables.

9. Dispositif de diagnostic à rayons X selon une des revendications 1 à 8, **caractérisé en ce qu'**une connexion réseau (10) est réalisée avec des composants standards pour une transmission de données sans fil.

10. Dispositif de diagnostic à rayons X selon une des revendications 1 à 9, **caractérisé en ce qu'**on utilise comme unité de calcul (8) un ordinateur portable ou un notebook.

11. Dispositif de diagnostic à rayons X selon une des revendications 2 à 10, **caractérisé en ce qu'**un moyen d'affichage (39) est associé à l'adaptateur mural (21) pour le contrôle des clichés qui précèdent.

12. Dispositif de diagnostic à rayons X selon la revendication 11, **caractérisé en ce que** le moyen d'affichage (39) est fixé dans l'adaptateur mural (21) de manière à pouvoir pivoter et/ou basculer.

13. Dispositif de diagnostic à rayons X selon la revendication 12, **caractérisé en ce que** le moyen d'affichage (39) forme avec un dispositif de déclenchement à distance (29) une unité de construction distincte de l'adaptateur mural (21).
